# EUROPEAN PATENT APPLICATION

(11) **EP 2 460 496 A1**
(43) Date of publication of application: **06.06.2012**
(21) Application number: 09847799.5
(22) Date of filing: 28.07.2009
(51) Int. Cl.: A61F 13/15, A61F 13/472

(54) **DISPOSABLE COMPOSITE PAD FOR BODILY FLUID**

(71) Applicant: Unicharm Corporation, Ehime 799-0111 (JP)
(72) Inventor: SUGA, Ayami, Kanonji-shi Kagawa 769-1602 (JP)
(74) Representative: Vaughan, Jennifer Ann
(86) International application number: PCT/JP2009/063443
(87) International publication number: WO 2011/013207

(57) **Abstract**

The present invention aims to provide a disposable bodily fluid absorbent composite pad allowing the wearer to recognize what number component pad is being used and how many useful component pads remain. A menstruation napkin 1 as a typical example of the bodily fluid absorbent composite pad includes a first component pad 10, a second component pad 20 and a third component pad 30 layered one on another. On the first component pad 10, two groups each consisting of three butterflies are drawn so as to be diagonally opposed to each other as indicator element 61. On the second component pad 20, two groups each consisting of two butterflies, i.e., reduced by one from each of the groups on the first component pad 10, are drawn as indicator element 62. On the third component pad 30, the total number of butterflies is further reduced to a single pair which is drawn as indicator element 63.

## Description

### TECHNICAL FIELD

The present invention generally relates to disposable bodily fluid absorbent composite pads including two or more component pads layered one on another and, more particularly, to such composite pads used, for example, as menstruation napkins, urine absorbent pads or panty liners.

### RELATED ART

Disposable bodily fluid absorbent composite pads including two or more component pads layered one on another are known. For example, PATENT CITATION 1 discloses such a composite pad including two or more layered liquid-absorbent component pads temporarily joined together along transversely opposite side edges by locally crimping these layered component pads. Each component pad may be peeled off one by one from a skin-facing side of the composite pad. More specifically, when the component pad contacting the wearer's skin is soiled, it may be peeled off from the remaining components to expose another fresh component pad.

### PRIOR ART DOCUMENT

### PATENT CITATION

PATENT CITATION 1: JP 4226086 B2

### SUMMARY OF THE INVENTION

### PROBLEM TO BE SOLVED BY THE INVENTION

In the course of using a composite pad comprising two or more component pads layered one on another, it becomes sometimes unclear for the wearer what number component pad is being used. When the last component pad is used and soiled, the composite pad must be exchanged with a fresh composite pad. However, if the wearer is unaware of the fact that the component pad being used is the last component pad, the wearer might fail to prepare a fresh composite pad and to exchange the used composite pad with the fresh one. Furthermore, the wearer sometimes deludes him/herself that the last component pad has been soiled and exchanges a composite pad still containing one or more useful component pads with the fresh composite pad. As a consequence, the remaining composite pad or pads might be vainly thrown away.

An object of the present invention is to provide a disposable bodily fluid absorbent composite pad allowing the wearer to recognize how many component pads remain.

### MEANS TO SOLVE THE PROBLEM

The present invention relates to an improvement of a disposable bodily fluid absorbent composite pad having a longitudinal direction and a transverse direction and including a skin-facing side, a non-skin-facing side opposite to the skin-facing side and two or more component pads each including an upper liquid-absorbent sheet on the skin-facing side and a lower liquid-impervious sheet on the non-skin-facing side and layered one on another.

The disposable bodily fluid absorbent composite pad according to the present invention is **characterized in that**:
the two or more component pads respectively include temporarily joined regions along which the component pads are temporarily joined so as to be peeled off from the other component pads and indicator elements adapted to be visually recognized from the skin-facing side; and
the indicator element displayed on one component pad is different from the indicator elements displayed on other component pads.

According to one embodiment of the present invention, the indicator element includes at least one of a letter, a graphic and a symbol as a constituent.

According to another embodiment of the present invention, the indicator element includes a numeric conception as a constituent.

According to yet another embodiment of the present invention, the indicator element includes a constituent having a series of concepts displayed depending on the order in which the component pads layered one on another.

According to still another embodiment of the present invention, the indicator element includes the same number of the letter or graphic or symbol as the number of the component pads counted from the non-skin-facing side.

According to further another embodiment of the present invention, the indicator element includes two or more of the graphics or symbols having a concept of temporal change and the graphics or symbols are differently displayed on each of the component pads so as to display the concept of temporal change corresponding to the order of lamination of the component pads.

According to another embodiment of the present invention, the temporarily joined regions are formed along a peripheral edge of the component pads by a heat crimping treatment, except at a partial segment of the peripheral edge treatment so that the partial segment left free from an effect of the heat crimping treatment serves as a guide region to indicate an appropriate region to be pinched by the wearer's fingers when it is desired that the soiled component pad is peeled off from the composite pad.

### EFFECT OF THE INVENTION

Each of two or more liquid-absorbent component pads layered one on another includes indicator an element adapted to be visually recognized from the skin-facing side and such indicator element on one component pad is different from the indicator element on an other component pad so that the individual component pads can be identified. The wearer can recognize what number component pad is being used and how many useful component pads remain on the basis of the particular pattern of the indicator element. In this way, the wearer can exchange the soiled composite pad with a fresh composite pad at the right time without waste.

### BRIEF DESCRIPTION OF THE DRAWINGS

[FIG. 1] Fig. 1 is a perspective view of a menstruation napkin.
[FIG. 2] Fig. 2 is a sectional view taken along line II-II in Fig. 1.
[FIG. 3] Fig. 3 is a schematic diagram illustrating Example 1.
[FIG. 4] Fig. 4 is a schematic diagram illustrating Example 2.
[FIG. 5] Fig. 5 is a schematic diagram illustrating Example 3.

### IDENTIFICATION OF REFERENCE NUMERALS USED IN THE DRAWINGS

- 1: menstruation napkin (disposable bodily fluid absorbent composite pad)
- 2A: guide region
- 10: first pad (pad)
- 11: liquid-absorbent sheet
- 12: liquid-impervious sheet
- 13: temporarily joined region
- 20: second pad (pad)
- 21: liquid-absorbent sheet
- 22: liquid-impervious sheet
- 23: temporarily joined region
- 30: third pad (pad)
- 31: liquid-absorbent sheet
- 32: liquid-impervious sheet
- 33: temporarily joined region
- 61-69: indicator elements
- X: transverse direction
- Y: longitudinal direction

### DETAILED DESCRIPTION OF THE PREFERRED EMBODIMENTS

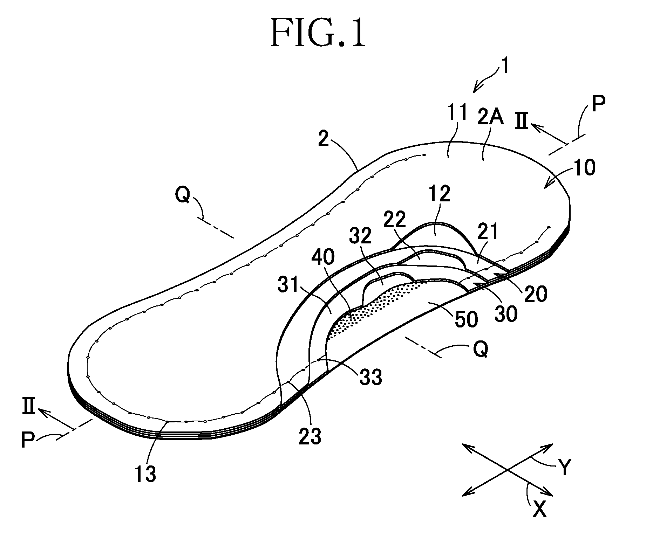

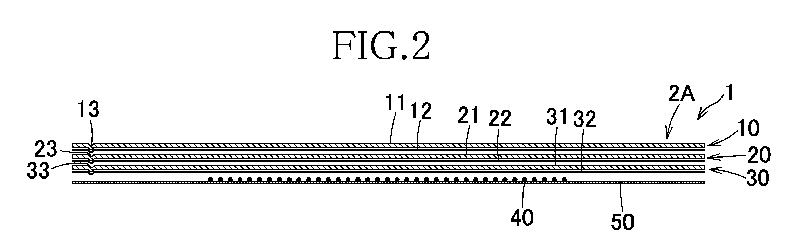

Details of a disposable bodily fluid absorbent composite pad according to the present invention will be described hereunder on the basis of a menstruation napkin as one typical embodiment of the invention. Construction of the menstruation napkin 1 shown in Figs. 1 and 2 is common to Examples 1 through 3. Fig. 1 is a perspective view showing the menstruation napkin 1 partially cutaway for convenience of illustration and Fig. 2 is a sectional view taken along line II-II in Fig. 1. The menstruation napkin 1 has a longitudinal direction Y and a transverse direction X wherein the menstruation napkin 1 is relatively long in the longitudinal direction Y and has a width gradually reduced from longitudinally opposite ends toward a central region. The menstruation napkin 1 has an imaginary longitudinal center line P-P bisecting a width dimension in the transverse direction X and an imaginary transverse center line Q-Q bisecting a length dimension in the longitudinal direction Y. The menstruation napkin 1 is shaped substantially symmetrically about the imaginary longitudinal center line P-P as well as about the imaginary transverse center line Q-Q.

The menstruation napkin 1 has a skin-facing side facing the wearer's body and a non-skin-facing side opposite to the skin-facing side and facing the wearer's garment. The menstruation napkin 1 includes two or more liquid-absorbent component pads layered from the skin-facing side to the non-skin-facing side. Specifically, the menstruation napkin 1 includes a first component pad 10 defining the uppermost layer of the composite pad, a second component pad 20 underlying the first component pad 10 and a third component pad 30 underlying the second component pad 20. These first, second and third component pads 10, 20, 30 respectively include upper liquid-absorbent sheets 11, 21, 31 and lower liquid-impervious sheets 12, 22, 32. The liquid-absorbent sheets 11, 21, 31 may be formed of a spun lace fibrous non-woven fabric made of a hydrophilic fiber such as cotton having a basis weight in a range of about 14 to 40g/m², preferably in a range of about 20 to 35g/m², more preferably of about 30g/m². The liquid-impervious sheets 12, 22, 32 may be formed of a plastic film having a basis weight in a range of about 10 to 25g/m², preferably of about 18g/m². The liquid-impervious sheets 12, 22, 32 preferably have breathability.

The non-skin-facing side, i.e., the side facing the wearer's garment of the third component pad 30 is formed with joining means 40 such as adhesive which is, in turn, covered from below with a releasable sheet 50. The releasable sheet 50 may be peeled off from the third pad 30 to expose the joining means 40 by the intermediary of which the third pad 30 may be fastened to the wearer's panty.

The first, second and third component pads 10, 20, 30 and the releasable sheet 50 are the same in shape as well as in size as one another and respectively have a dimension in the transverse direction X gradually reduced from longitudinally opposite ends toward a central region defined by the imaginary transverse center line Q-Q. By dimensioning the component pads 10, 20, 30 in this manner, it is assured that these component pads are put in close contact with the wearer's body without irregular deformation of these component pads in the crotch region.

The first, second and third component pads 10, 20, 30 are formed along the peripheral edge 2 thereof with individual temporarily joined regions 13, 23, 33. Specifically, these individual temporarily joined regions 13, 23, 33 include a plurality of depressions (debossings) arranged intermittently along the peripheral edge 2. The depressions are formed by collectively hot-pressing these first, second and third component pads 10, 20, 30 in the thickness direction so as to define a continuous linear temporarily joined region along which these component pads may be temporarily joined together. The joints between the temporarily joined regions 13, 23, 33 are easily peeled off by pulling the respective component pads 10, 20, 30 upwards so as to be spaced from one another. Each of the temporarily joined regions 13, 23, 33 is interrupted along a partial segment 2A of the peripheral edge 2 and such segments 2A serve as guide regions to guide the wearer to the appropriate end regions of the respective component pads 10, 20, 30 to be pinched with the fingers when it is desired to peel off the respective component pads 10, 20, 30 from the composite pad 1 and thereby to facilitate the end regions of the respective component pads 10, 20, 30 to be pinched with the fingers.

The first, second and third component pads 10, 20, 30 as have been described above are respectively formed with indicator elements which are peculiar to the respective component pads 10, 20, 30. The indicator elements may be formed directly on upper surfaces of the liquid-absorbent sheets 11, 21, 31 of the respective component pads 10, 20, 30 or on the liquid-impervious sheets 2, 22, 32 so as to be visually recognized through the associated liquid-absorbent sheets 11, 21, 31.

### EXAMPLE 1

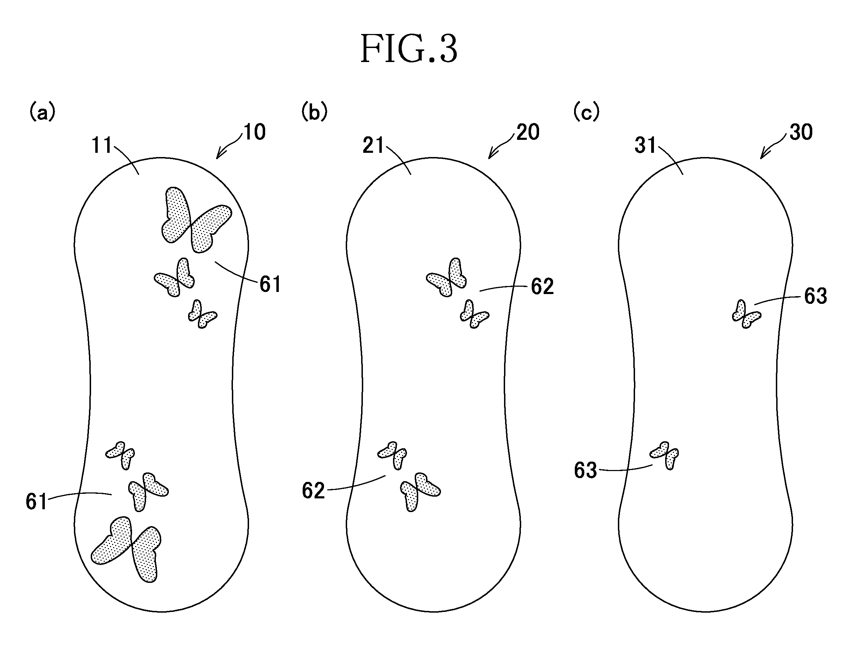

Fig. 3 illustrates indicator elements 61 through 63 used in Example 1 wherein graphics are used as the indicator elements. More specifically, FIG. 3 (a) illustrates the indicator elements 61 to be visually recognized in the first component pad 10, FIG. 3 (b) illustrates the indicator elements 62 to be visually recognized in the second component pad 20 and FIG. 3 (c) illustrates the indicator elements 63 to be visually recognized in the third component pad 30. On the first component pad 10, two groups of butterflies each consisting of three butterflies are drawn so as to be diagonally opposed to each other as indicator elements 61. On the second component pad 20, two groups each consisting of two butterflies, i.e., reduced by one from each of the groups on the first component pad 10, are drawn as indicator elements 62. On the third component pad 30, the total number of butterflies is further reduced to a single pair which is drawn as indicator elements 63. In this way, the wearer can easily know what number component pad is functioning or how many component pads are left to be used based on the number of butterflies drawn on the respective component pads as the indicator elements 61 through 63.

In this Example, if the last component pad, i.e., the third pad 30 is being used, a single pair of butterflies is displayed as the indicator elements 63 to indicate <this is the last component pad>. Even if it becomes unclear for the wearer what number component pad is being used, the wearer can understand that the last component pad is being used merely by counting the number of butterflies drawn on this component pad. Obviously the graphic used in the invention is not limited to the graphic of butterflies and it is possible to use the other various graphics such as a snow crystal mark or heart mark.

As has been described above, even if it becomes unclear for the wearer how many component pads are left to be useful and/or what number component pad is being used, the wearer can understand it merely by counting the number of the graphics displayed on the current component pad. In this way, it is possible for the wearer to exchange the used composite pad with a fresh composite pad at the right time and to avoid waste such as when a used composite pad still containing many useful component pads might be thrown away.

### EXAMPLE 2

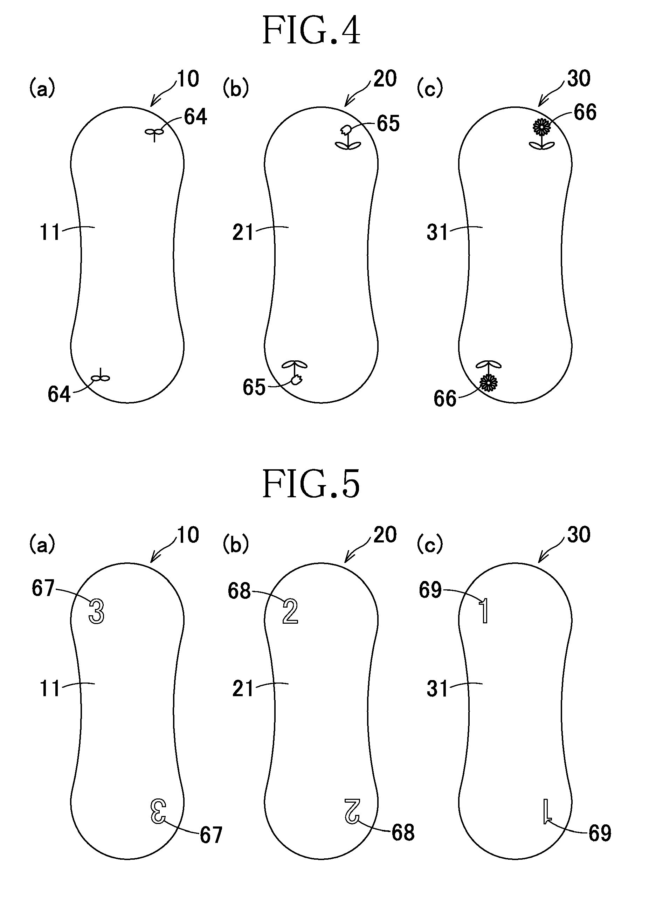

Fig. 4 is a view similar to Fig. 3, illustrating Example 2 wherein graphics are used as the indicator elements 64 through 66. The first component pad 10 includes a pair of green shoots drawn thereon as indicator elements 64, the second component pad 20 includes a pair of the green shoots grown-up to have blossom buds drawn thereon as the indicator elements 65 and the third component pad 30 includes a pair of the young shoots further grown-up to have flowers drawn thereon as indicator elements 66. These graphics represent a temporal change as a natural phenomenon. In general, a series of graphics which are suggestive of temporal change may be used as the indicator elements to assist the wearer to recognize what number component pad is being used.

Graphics being suggestive of a temporal change are generally indicated by a series of graphics allowing the wearer viewing one of these graphics to forecast the following graphic and thereby to conceive a series of temporal changes. For example, as a typical temporal change presented by astronomical objects, a series of graphics representing the temporal change starting from full moon to half-moon and then to crescent moon can be used. It is also possible to use a series of graphics representing a temporal change such that the sun sets and the moon rises. It is also possible to use a series of graphics representing a temporal change such that an apple is munched and gradually gets chipped.

### EXAMPLE 3

Fig. 5 is a view similar to Fig. 3, illustrating indicator elements 67 through 69 used in Example 3. The first component pad 10 includes a numeral "3" displayed thereon as the indicator element 67, the second component pad 20 includes a numeral "2" displayed thereon as the indicator element 68 and the third component pad 30 includes a numeral "1" displayed thereon as the indicator element 69. In this manner, the remaining number of the component pads is directly displayed on the respective component pads and allow the wearer to recognize the number of remaining useful component pads. While the numeral "1" is displayed on the third component pad 30 to tell the wearer that this component pad 30 is a useful last component pad, it is also possible to display a numeral "0" on the third component pad 30 and thereby to suggest there is no more useful component pad.

These numerals may be replaced by letters having any concept of sequential as the indicator elements. "A", "B", "C" of the alphabet is a typical example of such letters having the concept of sequential.

In the respective Examples 1 through 3, the indicator element may be formed not only by printing or the like technique but also by locally embossing the surfaces of the respective component pads. In any case, it is essential that these indicator elements can be visually recognized from the side of the liquid-absorbent sheets 11, 21, 31 of the component pads 10, 20, 30. While the respective Examples have been described on the assumption that each of them is a trilaminar composite pad, the invention is not limited to a trilaminar composite pad and the number of the component pads may be optional provided the number of the component pads is two or more.

While the indicator elements 61 through 69 have been described to be displayed symmetrically about the imaginary longitudinal center line P-P as well as about the imaginary transverse center line Q-Q, the invention is not limited to this. For example, the indicator element(s) may be displayed at a single position. When the indicator element(s) is or are displayed only one of the opposite ends in the longitudinal direction Y, the wearer can recognize the orientation of the composite pad during its use on the basis of the position at which the indicator element(s) is or are displayed.

In the lowermost third component pad 30, a fibrous non-woven fabric such as a spun bond/melt blown/spun bond (SMS) non-woven fabric may be additionally layered on the lower surface of the third component pad 30 and the joining means 40 is provided on this fibrous non-woven fabric. The non-woven fabric additionally layered on the third component pad 30 serves as a reinforcing member preventing the third component pad 30 from being damaged in the course of peeling off the third component pad 30 from the wearer's garment such as panty for the purpose of exchanging the composite pad 1. By using the fibrous non-woven fabric as the reinforcing member, stiffness of the liquid-impervious sheet 32 constituting the third component pad 30 can be reduced and thereby feeling of discomfort which might otherwise be experienced by the wearer during use of the composite pad 1 can be alleviated.

## Claims

1. A disposable bodily fluid absorbent composite pad having a longitudinal direction and a transverse direction and comprising a skin-facing side, a non-skin-facing side opposite to the skin-facing side and two or more component pads each including an upper liquid-absorbent sheet on the skin-facing side and a lower liquid-impervious sheet on the non-skin-facing side and layered one on another, wherein:
the two or more component pads respectively include temporarily joined regions along which the component pads are temporarily joined so as to be peeled off from the other component pads and indicator elements adapted to be visually recognized from the skin-facing side; and
the indicator element displayed on one component pad is different from the indicator elements displayed on other component pads.

2. The disposable bodily fluid absorbent composite pad defined by Claim 1, wherein the indicator element includes at least one of a letter, a graphic and a symbol as a constituent.

3. The disposable bodily fluid absorbent composite pad defined by Claim 2, wherein the indicator element includes a numeric concept as the constituent.

4. The disposable bodily fluid absorbent composite pad defined by Claim 2 or 3, wherein the indicator element includes a constituent having a series of concepts displayed depending on the order in which the component pads are layered one on another.

5. The disposable bodily fluid absorbent composite pad defined by any one of Claims 2 through 4, wherein the indicator element comprises the same number of the letter or graphic or symbol as the number of the component pads counted from the non-skin-facing side.

6. The disposable bodily fluid absorbent composite pad defined by Claim 2, wherein the indicator element comprises two or more of the graphics or symbols having a concept of temporal change and the graphics or symbols are differently displayed on each of the component pads so as to display the concept of temporal change corresponding to the order of lamination of the component pads.

7. The disposable bodily fluid absorbent composite pad defined by any one of Claims 1 through 5, wherein the temporarily joined regions are formed along a peripheral edge of the component pads except at a partial segment of the peripheral edge by a heat crimping treatment so that the partial segment left free from an effect of the heat crimping treatment serves as a guide region to indicate an appropriate region to be pinched by the wearer's fingers when it is desired that the soiled component pad is peeled off from the composite pad.
